# EUROPEAN PATENT SPECIFICATION

(11) **EP 2 594 255 B1**
(45) Date of publication and mention of the grant of the patent: **08.05.2019**
(21) Application number: 11806740.4
(22) Date of filing: 11.07.2011
(51) Int. Cl.: A61K 8/34, A61K 8/44, A61Q 11/00

(54) **DENTIFRICE COMPOSITION**
ZAHNPASTAZUSAMMENSETZUNG
COMPOSITION DE PÂTE DENTIFRICE

(30) Priority: 12.07.2010 JP 2010157524
(43) Date of publication of application: 22.05.2013
(73) Proprietor: Kao Corporation, Chuo-Ku Tokyo 103-8210 (JP)
(72) Inventor: TAKAHASHI, Noritaka, Tokyo 131-8501 (JP)
(74) Representative: Hoffmann Eitle
(86) International application number: PCT/JP2011/065789
(87) International publication number: WO 2012/008410

(56) References cited:
- EP-A2- 0 364 928
- JP-A- 10 306 018
- JP-A- 2005 029 484
- JP-A- 2006 176 463
- JP-A- 2008 013 528
- KAUKO K. MÄKINEN: "Sugar Alcohols, Caries Incidence, and Remineralization of Caries Lesions: A Literature Review", INTERNATIONAL JOURNAL OF DENTISTRY, vol. 58, no. 1, 1 January 2010 (2010-01-01), pages 332-23, XP055096136, ISSN: 1687-8728, DOI: 10.1159/000189703
- DATABASE CA [Online] CHEMICAL ABSTRACTS SERVICE, COLUMBUS, OHIO, US; 24 January 2008 (2008-01-24), MAEDA, AKITSUGU ET AL: "Dentifrice compositions containing erythritol and salt with specified particle sizes, and manufacture thereof", XP002744054, retrieved from STN Database accession no. 2008:91806

## Description

### Field of the Invention

The present invention relates to a dentifrice composition.

### Background of the Invention

To prevent oral diseases, such as dental caries, periodontal disease and halitosis, it is important to inhibit the formation of a biofilm in the oral cavity and to remove the biofilm already formed in the oral cavity. Erythritol and amino acid have conventionally been known as active ingredients for removing the biofilm (Patent Document 1). With regard to erythritol, a dentifrice containing a large amount (30 to 60 mass%) of particles of erythritol, is proposed from the viewpoint of sufficiently achieving a refreshing feel caused by endothermic reaction in its dissolution (Patent Document 2) .

On the other hand, with regard to N-acyl amino acid, there are known technologies for suppressing formation of plaque by using N-acyl amino acid together with a cationic bactericide, triclosan, or a biguanide bactericide to promote adsorbability of the bactericide on surfaces of teeth or to suppress deactivation of the bactericide (Patent Documents 3 to 5). N-acyl amino acid is further known to have an effect of improving foaming property, and Patent Document 6 discloses that N-long chain acyl amino acid or a salt thereof is blended to compensate foaming property of polyoxyethylene stearyl alcohol ether, thereby improving foaming property of a toothpaste.

International journal of dentistry, volume 2010, pp. 323-332, provides a literature review in regard to the relationship between sugar alcohols, caries incidence, and remineralization of caries lesions.

EP 0 364 928 A2 relates to a paste-like dentifrice composition containing, as the foaming agent, from 0.1 to 5.0% by weight of highly pure N-long-chain acylglutamate which contains not more than 1.0% by weight of higher fatty acid(s), either in the free form or in the salt form.

JP 2008-013528 A relates to a dentifrice composition comprising (A) 15-60 mass% of erythritol having a particle diameter of ≥45*µ*m and < 355*µ*m, (B) 3-35 mass% of salt having a particle diameter of ≥500*µ*m and < 1,000*µ*m, (C) 0.3-10 mass% of a binder and (D) 10-30 mass% of water and has the mass ratio of salt to erythritol of ≤1.

JP 2005-029484 A relates to a composition for the oral cavities containing a sugar alcohol and/or an amino acid. The composition is said to be able to control the adhesion and coagulation of bacteria and to degrade the formed adhesion products and cocoagulated products to inhibit the formation of the biofilms, in a process for forming the biofilms in spaces, such as periodontal pockets, between hard tissues, such as teeth or artificial teeth, and soft tissues, such as tongues or cheeks, and to further be able to change the inner structures of the already matured biofilms to promote the removal of the biofilms.

JP 10-306018 A relates to an agent for improving the activity of enzyme for oral cavity and can be produced by including a component selected from (A) a nonionic surfactant (e.g. decaglyceryl monomyristate), (B) an amino acid-based anionic surfactant (e.g. lauroylmethyl-*β*-alanine sodium), (C) a polyethylene glycol having an average molecular weight of preferably ≤1, 000, (D) a polymeric polysaccharide (e.g. pectin) and (E) a sugar alcohol (e.g. glycerol).

### Citation List

### Patent Document

[Patent Document 1] JP-A-2005-029484
[Patent Document 2] JP-A-2005-281306
[Patent Document 3] JP-A-4-036231
[Patent Document 4] JP-A-2-001402
[Patent Document 5] JP-A-2-282317
[Patent Document 6] JP-A-53-091147

### Summary of the Invention

The present invention provides a dentifrice composition, comprising the following components (A), (B), and (C) :
(A) 10 to 65 mass% of erythritol;
(B) 0.05 to 0.55 mass% of an N-acyl acidic amino acid or a salt thereof; and
(C) 10 to 25 mass% of water,
wherein the mass ratio (A/B) of the component (A) to the component (B) is 20 to 400, the content of sodium chloride in the dentrifice composition is 0.1 mass% or less, and an acyl group constituting the N-acyl acidic amino acid as the component (B) has 8 to 16 carbon atoms.

The present invention also provides for a use of the dentifrice composition in a method of removing a biofilm in the oral cavity.

### Brief Description of the Drawings

[FIG. 1] A graph showing results of a biofilm removal test.
[FIG. 2] A graph showing the results of a biofilm removal test.

### Detailed Description of the Invention

Biofilm in the oral cavity of humans is composed of a co-aggregate of various oral bacteria and extracellular polysaccharides, and removal of biofilm in the oral cavity once formed is not always easy. As mentioned in Patent Documents 3 to 5, prevention of deactivation of a bactericide and promotion of adsorption of the bactericide to teeth are effective for prevention of the formation of biofilm. However, the bactericide applicable to the oral cavity is not effective on the oral bacteria in biofilm already formed and therefore it is difficult to remove the biofilm.

On the other hand, erythritol is known to have a biofilm-removing effect but is desired to have a better effect.

Therefore, the present invention relates to a dentifrice composition which contains erythritol and has an improved effect of removing an already formed biofilm.

The inventor of the present invention searched a component for improving the biofilm-removing effect provided by erythritol, and as a result, found that the biofilm-removing effect is significantly improved by incorporating N-acyl acidic amino acid among N-acyl amino acids together with erythritol at a particular ratio.

The dentifrice composition of the present invention contains erythritol and has a significantly improved biofilm-removing effect provided by erythritol in the oral cavity.

Preferred embodiments of the present invention include those as shown below.
[1] A dentifrice composition, comprising the following components (A), (B), and (C) :
   (A) 10 to 65 mass% of erythritol;
   (B) 0.05 to 0.55 mass% of an N-acyl acidic amino acid or a salt thereof; and
   (C) 10 to 25 mass% of water,
   wherein the mass ratio (A/B) of the component (A) to the component (B) is 20 to 400, the content of sodium chloride in the dentrifice composition is 0.1 mass% or less and an acyl group that constitutes the N-acyl acidic amino acid as the component (B) has 8 to 16 carbon atoms.
[2] The dentifrice composition according to the item [1], wherein the mass ratio (A/C) of the component (A) to the component (C) is 0.7 to 6.
[3] The dentifrice composition according to the item [1] or [2], wherein an acidic amino acid that constitutes the N-acyl acidic amino acid as the component (B) is glutamic acid.
[4] The dentifrice composition according to any one of the items [1] to [3], further comprising 0.1 to 2.0 mass% of an alkyl sulfate salt as a component (D), wherein the mass ratio (D/B) of the component (D) to the component (B) is 0.2 to 20.
[5] The dentifrice composition according to any one of the items [1] to [4], wherein the component (A) comprises 10 to 55 mass% of particulate erythritol having an average particle size of 350 µm or less.
[6] The dentifrice composition according to any one of the items [1] to [5], wherein the mass ratio (A/B) of the component (A) to the component (B) is 20 to 250.
[7] The dentifrice composition according to any one of the items [1] to [6], wherein the mass ratio (A/C) of the component (A) to the component (C) is 0.9 to 4.
[8] The dentifrice composition according to any one of the items [1] to [7], wherein the mass ratio (D/B) of the component (D) to the component (B) is 2 to 15.
[9] The dentifrice composition according to any one of the items [1] to [8], wherein the content of the water as the component (C) is 10 to 20 mass%.
[10] The dentifrice composition according to any one of the items [1] to [9], further comprising 0 to 35 mass%, preferably 10 to 24.5 mass% of sorbitol and/or maltitol.
[11] The dentifrice composition according to the items [1] to [10], further comprising 0 to 8 mass%, preferably 1 to 6 mass% of glycerin.
[12] The dentifrice composition according to any one of the items [1] to [11], wherein the content of sodium chloride in the dentrifice composition is 0.01 mass% or less.
[13] The dentifrice composition according to any one of the items [1] to [12] for use in a method of removing a biofilm in the oral cavity.

Hereinafter, the present invention is described.

The dentifrice composition of the present invention contains 10 to 65 mass% of erythritol (component (A)). In view of achieving a biofilm-removing effect and a refreshing feel, the content of the erythritol (A) is more preferably 10 to 55 mass%, and even more preferably 10 to 50 mass% in the dentifrice composition.

From the viewpoint of achieving a persistent refreshing feel caused by negative heat of solution, the erythritol (A) of the present invention preferably contains particulate erythritol, and more preferably erythritol having an average particle size of 350 µm or less. In view of achieving a good refreshing feel, the average particle size of erythritol is preferably 250 µm or less, and more preferably 200 µm or less. The content of particulate erythritol in the dentifrice composition of the present invention is preferably 10 to 55 mass%, more preferably 10 to 45 mass%, and even more preferably 10 to 40 mass%.

It should be noted that the particle size of erythritol is determined as described below.
Sieve: JIS Standard Sieve ϕ75 mm
Opening: A receiver is placed below sieves having openings of 500 pm, 355 µm, 250 µm, 180 µm, 125 µm, 90 µm, and 45 µm in this order from the top stage.
Shaker: Micro electromagnetic shaker Model M-2 (Tsutsui Scientific Instruments Co., Ltd.)
Method: On the 500 µm sieve is placed 15 g of a sample, followed by classification using the electromagnetic shaker for 5 minutes. Ratios of particles that have passed through the sieve (cumulative amounts) were plotted on normal probability paper, and a value corresponding to 50% was defined as an average particle size.

Such particle size can be adjusted by pulverizing crystalline erythritol. Pulverization of erythritol may be carried out by using a roller mill, a hammer mill, a high-speed pulverizer, or a pulverizer. Examples of crystalline erythritol include commercially available products manufactured by NIKKEN CHEMICAL AND SYNTHETIC INDUSTRY CO., LTD., Mitsubishi-Kagaku Foods Corporation, and Cargill.

The dentifrice composition of the present invention further contains N-acyl acidic amino acid or a salt thereof (component (B)). In view of foaming property and foam quality, the acyl group constituting the N-acyl acidic amino acid has 8 to 16 carbon atoms, and the acyl group is more preferably one kind or two or more kinds selected from a myristoyl group and a lauroyl group. Examples of the acidic amino acid constituting the N-acyl acidic amino acid include glutamic acid and aspartic acid, and glutamic acid is preferred. Examples of the salt of the N-acyl acidic amino acid include a sodium salt, a potassium salt, and a magnesium salt, and a sodium salt is preferred.

In view of a balance between a defoaming treatment in production of the dentifrice composition and foam quality, the content of the N-acyl acidic amino acid or salt thereof (B) in the dentifrice composition of the present invention is 0.05 to 0.55 mass%, preferably 0.1 to 0.5 mass%, and more preferably 0.1 to 0.4 mass%. In view of a biofilm-removing effect and a balance between defoaming property and foam quality, the mass ratio (A/B) of the content of the erythritol (A) to the content of the N-acyl acidic amino acid (B) in the dentifrice composition is preferably 20 to 400, more preferably 20 to 300, and even more preferably 20 to 250.

The dentifrice composition of the present invention further contains water (component (C)). The water (C) includes not only purified water and ion exchanged water to be blended but also water in the dentifrice composition, which includes water in each component, such as water in a sorbitol solution. From the viewpoints of producing a composition containing the erythritol (A) in a particle (including powder) form although part of the erythritol (A) is dissolved, and achieving a refreshing feel caused by dissolution of erythritol when used, and in view of achieving good stability, the content of water in the dentifrice composition is 10 to 25 mass%, and preferably 10 to 20 mass%. The mass ratio (A/C) of the content of the erythritol (A) to the content of the water (C) is preferably 0.7 to 6, more preferably 0.8 to 5, and even more preferably 0.9 to 4.

When the content of the water (C) in the dentifrice composition of the present invention is 10 to 25 mass% , the solubility of the erythritol (A) at 20°C is 33%, and hence erythritol (A1) to be dissolved in water is 6.6 to 16 . 5 mass% . The mass ratio (A1/B) of the erythritol (A1) in the dissolved state to the N-acyl acidic amino acid (B) is preferably 15 to 300, more preferably 20 to 150, and even more preferably 20 to 110.

In this respect, the amount of water in the dentifrice composition can be calculated from the amount of blended water and the amount of water in a blended component, but can be measured by, for example, a Karl Fischer moisture meter. As the Karl Fischer moisture meter, for example, a trace moisture meter (Hiranuma Sangyo Corporation) may be used. In the device, 5 g of the dentifrice composition was suspended in 25 g of anhydrous methanol, and 0.02 g of the suspension was separated and used in measurement of the amount of water.

In view of preventing a reduction in the biofilm-removing effect provided by the components (A) to (C), the dentifrice composition of the present invention is preferably substantially free of sodium chloride . The "substantially free of sodium chloride" as used herein refers to a state where the content of sodium chloride in the dentifrice composition is 0.1 mass% or less, and the content is preferably 0.05 mass% or less, more preferably 0.01 mass% or less, and even more preferably 0.

The dentifrice composition of the present invention preferably further contains an alkyl sulfate salt (D). In view of foaming property, the alkyl sulfate salt is preferably lauryl sulfate or myristyl sulfate, and the salt thereof is preferably a sodium salt. The content of the alkyl sulfate salt (D) is preferably 0.1 to 2 mass%, more preferably 0.25 to 1.75 mass%, and even more preferably 0.5 to 1. 5 mass% . In view of achieving a balance between foaming property or foam quality and defoaming property, the mass ratio (D/B) of the alkyl sulfate salt (D) to the N-acyl acidic amino acid (B) in the dentifrice composition is preferably 0.2 to 20, more preferably 1 to 15, and even more preferably 2 to 15.

The dentifrice composition of the present invention may contain a sugar alcohol other than the erythritol (A). Examples of the sugar alcohol include sorbitol, xylitol, maltitol, mannitol, and palatinit. In view of defoaming property, in the case where the composition contains sorbitol and/or maltitol, the content of a sorbitol solution (70% aqueous solution) and/or a maltitol solution (70% aqueous solution) in the dentifrice composition is preferably 50 mass% or less, more preferably 40 mass% or less, and even more preferably 35 mass% or less. That is, the content of sorbitol and/or maltitol in the dentifrice composition is preferably 0 to 35 mass%, more preferably 10 to 28 mass%, and even more preferably 10 to 24.5 mass%.

The dentifrice composition of the present invention may contain a wetting agent. Examples of the wetting agent include polyethylene glycol, propylene glycol, dipropylene glycol, and glycerin. The wetting agent may be contained in the dentifrice composition in an amount of 0 to 15 mass%. In view of defoaming property, the content of glycerin in the dentifrice composition is preferably 0 to 8 mass%, and more preferably 1 to 6 mass%.

The dentifrice composition of the present invention preferably further contains a binder. As the binder, there may be used one kind, or two or more kinds of binders selected from the group consisting of sodium alginate, sodium carboxymethylcellulose, carrageenan, xanthan gum, sodium polyacrylate, hydroxyethyl cellulose, hydroxypropyl cellulose, pectin, tragacanth gum, acacia gum, guar gum, karaya gum, locust bean gum, gellan gum, tamarind gum, psyllium seed gum, polyvinyl alcohol, sodium chondroitin sulfate, a methoxyethylene-maleic anhydride copolymer, and the like. Of those, sodium carboxymethylcellulose, carrageenan, and xanthan gum are preferred, and carrageenan and sodium carboxymethylcellulose are more preferred. In view of shape retention property, stickiness, and feeling, using two or more kinds of binders are preferred, and using three or more kinds of binders are more preferred. The content of the binder in the dentifrice composition is preferably 0.1 to 3 mass%, and more preferably 0.1 to 2 mass%. The content is also preferably in the range of 0.6 to 3 mass%, more preferably 0.5 to 2.5 mass%.

The dentifrice composition of the present invention preferably further contains thickening silica together with the binder. The thickening silica has the capacity of oil absorption of from 200 to 400 mL/100 g. The "oil absorption" as used herein refers to the amount of an oil which can be supported by silica and is determined by a method according to JIS K5101-13-2 based on the amount of boiled linseed oil to be absorbed. The content of the thickening silica in the dentifrice composition is preferably 0.5 to 10 mass%.

In addition to the above-mentioned components, a polishing material, a sweetening agent, a flavoring agent, a pH adjusting agent, a dye, or a medicinal component may be appropriately incorporated into the dentifrice composition of the present invention.

Examples of the polishing material include abrasive silica, dicalcium phosphate dihydrate and anhydride, calcium pyrophosphate, calcium carbonate, alumina, aluminum hydroxide, magnesium acetate, dimagnesium phosphate, magnesium acetate, trimagnesium phosphate, zeolite, and a synthetic resin polishing material. Herein, the abrasive silica has the capacity of oil absorption of from 50 to 150 mL/100 g.

Examples of the sweetening agent include saccharin sodium, aspartame, thaumatin, acesulfame potassium, stevioside, stevia extract, p-methoxycinnamic aldehyde, neohesperidyl dihydrochalcone, perillartine, and sucralose.

Examples of the flavoring agent include 1-menthol, carvone, anethole, eugenol, limonene, peppermint oil, spearmint oil, ocimene, n-amyl alcohol, citronellol, α-terpineol, methyl salicylate, methyl acetate, citronellylacetate, cineole, linalool, ethyllinalool, vanillin, thymol, lemon oil, orange oil, sage oil, rosemary oil, cinnamon oil, pimento oil, shiso oil, clove oil, and eucalyptus oil.

Examples of the medicinal component include a bactericide, an anti-inflammatory agent, a blood flow-improving agent, and a fluorine ion-feeding compound. Examples of the bactericide include chlorhexidines, quaternary ammonium compounds such as cetylpyridinium chloride and benzethonium chloride, isopropylmethylphenol, triclosan, and trichlorocarbanilide. Examples of the fluorine ion-feeding compound include inorganic fluorides such as sodium fluoride, potassium fluoride, ammonium fluoride, lithium fluoride, monofluorophosphates (e.g., sodium monofluorophosphate, potassium monofluorophosphate, and ammonium monofluorophosphate), and organic fluorides such as an amine fluoride. Of those, sodium fluoride, sodium monofluorophosphate, and tin fluoride are preferred.

In view of stability of the N-acyl acidic amino acid, the dentifrice composition of the present invention has a pH of preferably 5 to 9, and more preferably 6 to 8. In this case, the pH of the dentifrice composition of the present invention can be measured by adding distilled water to the composition to prepare a 10 mass% aqueous solution and subsequently measuring the pH using a pH electrode.

The dentifrice composition of the present invention is preferably a paste dentifrice composition, and from the viewpoint of achieving a sufficient refreshing feel, erythritol having a particular particle size is desirably dispersed in a particle (including powder) form. For that purpose, it is preferred that erythritol is introduced directly in the form of a powder in the final step of production. The use of suchproduction process enables erythritol to be present in a particulate form in the dentifrice composition. Specifically, for example, components such as purified water, a wetting agent, a binder, a preservative, a sweetening agent, and a medicinal ingredient can be mixed according to given conditions of production to sufficiently swell the binder, followed by adding and mixing a polishing agent, thickening silica, N-acyl acidic amino acid, and if necessary, another surfactant and a flavoring agent. Finally, particulate erythritol is added thereto for defoaming mixing to produce the dentifrice composition of the present invention.

### Examples

Hereinafter, the present invention is described more specifically by way of examples and test examples. However, the present invention is not limited to the examples. In this regard, in the formulations shown in the tables, the contents of the components represent percentages by mass unless otherwise specified.

### (1) Preparation of biofilm

First 50 mL of a culture solution (SCD-YE medium) obtained by adding 0.5% yeast extract (Difco) to SCD medium (soybean-casein digest broth; NIHON PHARMACEUTICAL CO., LTD.). Next each bacterial strain of *Streptococcus mutans* ATCC 25175, *Actinomyces viscosus*ATCC 43146, and *Streptococcus gordonii* ATCC 10558 was inoculated by inoculation loop in the culture solution and cultured at a temperature of 37°C under anaerobic conditions (80% N₂, 20% CO₂) until a logarithmic growth phase was achieved.

The saliva secreted in response to chewing of paraffin wax from a healthy man who does not suffer from oral disease was collected and centrifuged to remove peeled oral mucosa or the like from the saliva. The resultant was dispensed in glass base dishes (IWAKI ϕ27 mm) in an amount of 2 mL/dish and allowed to stand still overnight at 4°C to adsorb the human saliva on the glass base dishes.

The bacteria cultured above (*S. mutans, A. viscosus,* and *S. Gordonii*) were adjusted with a culture solution (SCD-YE medium) so as to have O.D. of 0.7 determined at 600 nm with an optical path length of 10 mm by a spectrophotometer (BioSpec-mini; Shimadzu Corporation), and 1 mL of each bacteria was inoculated into 50 mL of the culture solution to prepare a bacteria mixed solution. The resultant solutions were added to the glass base dishes to which the saliva had been adhered in an amount of 2 mL/dish, and the bacteria were cultured for 24 hours to form biofilms. In this case, a culture supplemented with 0.5% of yeast (yeast extract; Difco), 1.0% of glucose, and 2.0% of sucrose was used as the culture.

### (2) Biofilm removal test

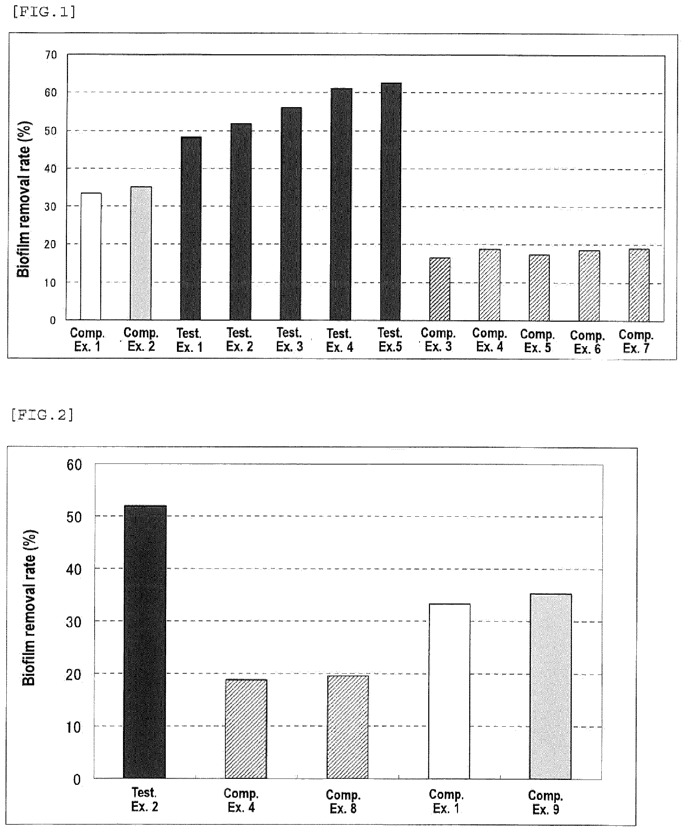

The compositions shown in Table 1 (Test Examples 1 to 5 and Comparative Examples 1 to 10) were added dropwise in an amount of 1 mL to the biofilms prepared in (1), followed by ultrasonication at room temperature (20°C) for 30 seconds. The ultrasonication in the removal test was carried out using an ultrasonic washing machine AW5800 (CITIZEN SYSTEMS JAPAN CO. LTD.) designed to give an ultrasonic frequency of 42 KHz, which is similar to human's gargle. After the treatment, the amount of peeled biofilm (A) and the amount of residual biofilm (B) were measured by a phenol-sulfuric acid method. The residual biofilm was peeled off by adding 1 mL of 2 N sodium hydroxide, and 1 mL of 2 N hydrochloric acid was further added to neutralize the mixture, followed by collection. The peeled biofilm was collected by adding 1 mL of purified water together with 1 mL of the composition. The amount of the collected biofilm was measured by the phenol-sulfuric acid method. The phenol-sulfuric acid method was carried out by: dispensing 20 µL of the collected biofilm to a 96-well microplate ; adding 20 µL of a 5%(W/V) aqueous phenol solution; adding 100 µL of 98% concentrated sulfuric acid; mixing the solution; and measuring an absorbance at 490 nm using the microplate reader. A calibration curve was created by determining the absorbances of 0.0078 to 0.125% glucose solutions to quantify the amounts of biofilms. The biofilm removal rate was calculated from the following expression: amount of biofilm (A)/(amount of biofilm (A) +amount of biofilm (B)) . Table 1 and FIGS. 1 and 2 show the test results. In this regard, in the compositions shown in Table 1, erythritol having an average particle size of 200 µm was blended and dissolved completely, and the biofilm removal test was carried out.

**[Table 1]**

| | Test Example 1 | Test Example 2 | Test Example 3 | Test Example 4 | Test Example 5 | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|---|---|---|
| Sodium N-myristoyl-L-glutamate (*1) | 0.1 | 0.2 | 0.3 | 0.4 | 0.5 | 0 | 0.02 | 0.1 | 0.2 | 0.3 | 0.4 | 0.5 | 0.2 | - | 0.2 |
| sodium N-cocoyl glycinate (*2) | - | - | _ | - | - | - | - | - | - | - | - | - | - | 0.2 | - |
| Erythritol (*3) | 10 | 10 | 10 | 10 | 10 | 10 | 10 | - | - | - | - | - | - | 10 | 10 |
| Sorbitol | - | - | - | - | - | - | - | - | - | - | - | - | 10 | - | - |
| Sodium chloride | - | - | - | - | - | - | - | - | - | - | - | - | - | - | 10 |
| Water | Balance | Balance | Balance | Balance | Balance | Balance | Balance | Balance | Balance | Balance | Balance | Balance | Balance | Balance | Balance |
| Total | 100 | 100 | 100 | 100 | 100 | 100 | 100 | 100 | 100 | 100 | 100 | 100 | 100 | 100 | 100 |
| Biofilm removal rate (%) | 48.25 | 51.88 | 56.04 | 62.18 | 62.53 | 33.38 | 35.21 | 16.44 | 18.61 | 17.42 | 18.69 | 19.04 | 19.59 | 35.3 | 35.49 |

| | | | | | | | | | | | | | | | |
|---|---|---|---|---|---|---|---|---|---|---|---|---|---|---|---|
| (*1) AMISOFT MS-11 (AJINOMOTO CO., INC.) (*2) AMILITE GCS-11 (AJINOMOTO CO., INC.) (*3) Average particle size: 200 µm | | | | | | | | | | | | | | | |

As shown in Table 1 and FIGS. 1 and 2, the compositions of Comparative Example 3 to 7 containing only sodium N-myristoyl glutamate had low biofilm removal rates regardless of the content of sodiumN-myristoyl glutamate. On the other hand, the compositions of Test Examples 1 to 5 containing erythritol and sodium N-myristoyl glutamate at mass ratios ranging from 20 to 400 had high biofilm-removing effects compared with the composition of Comparative Example 1 containing only erythritol and the composition of Comparative Example 2 containing erythritol and sodium N-myristoyl glutamate at a mass ratio of 500. Further, as shown in Table 1 and FIGS. 1 and 2, the composition of Comparative Example 8 containing sorbitol and sodium N-myristoyl glutamate had a biofilm removal rate similar to that of the composition of Comparative Example 4 containing only sodium N-myristoyl glutamate, which suggested that the biofilm-removing effect was not improved even when the composition contained the N-acyl acidic amino acid together with sorbitol. In addition, the composition of Comparative Example 9 containing sodium N-cocoyl glycinate which is the N-acyl neutral amino acid had a biofilm removal rate similar to that of the composition of Comparative Example 1 containing only erythritol, which suggested that the biofilm-removing effect was not improved even when the composition contained the N-acyl neutral amino acid together with erythritol. The dentifrice composition of Comparative Example 10 containing sodium chloride had a lowered biofilm-removing effect.

### (3) Foam quality and defoaming property

The dentifrice compositions shown in Table 2 were evaluated for the foam quality when the dentifrice compositions were used and the defoaming property when components in the dentifrice compositions were mixed. In this regard, the pH of the dentifrice compositions was adjusted to 6.5 to 7.5.

The foam quality of the dentifrice composition was evaluated by three persons using the composition of Comparative Example 11 as a standard. When the foam is finer than, equal to, and coarser than that of Comparative Example 11, the composition was evaluated to be 1, 0, and -1, respectively. When the foam retention property is better than, equal to, and poorer than that of Comparative Example 11, the composition was evaluated to be 1, 0, and -1, respectively. The evaluations were made based on discussions by the three persons . Each dentifrice composition was used for evaluations by placing 1.5 cm of the composition on a toothbrush (Puora regular size (Kao Corporation)) and brushing teeth with the toothbrush for 1 minute.

The defoaming property was evaluated as follows. Each of the compositions shown in Table 2 was stirred at a rotation frequency of 8 rpm and a vacuum pressure of 0.09 mPa for 10 minutes, and the specific gravity (specific gravity 1) was measured. Subsequently, the composition was stirred at a rotation frequency of 8 rpm and a vacuum pressure of 0.096 mPa for 15 minutes, and the specific gravity (specific gravity 2) was measured. The ratio of the specific gravity 2/specific gravity 1 was calculated to evaluate the defoaming property. The case where the composition had a specific gravity ratio of 0.98 or less because of a decreased specific gravity was evaluated "poor" and the case where the composition had a specific gravity ratio of 1.00 to 0.98 was evaluated "good". In this case, as a stirring and mixing machine, a universal stirring and mixing machine NDM/NDMV manufactured by DALTON was used.

**[Table 2]**

| | Example 1 | Example 2 | Example 3 | Example 4 | Comparative Example 11 | Comparative Example 12 | Comparative Example 13 | Comparative Example 14 | Comparative Example 15 |
|---|---|---|---|---|---|---|---|---|---|
| Purified water | 6.8 | 6.5 | 6.9 | 6.8 | 7 | 6.8 | 6.3 | 6.9 | 6.9 |
| Sodium lauryl sulfate (D) | 1.5 | 1.5 | 1.5 | 1.5 | 1.5 | 1.5 | 1.5 | 1.5 | 1.5 |
| Sodium N-myristoyl-L-gluta mate (*1) (B) | 0.2 | 0.5 | 0.1 | 0.2 | 0 | 0.2 | 0.7 | 0.1 | 0.3 |
| Sorbit solution (70%) | 30 | 30 | 30 | 20 | 30 | 60 | 30 | 20 | 25.5 |
| Glycerin (98%) | 5 | 5 | 5 | 5 | 5 | 10 | 5 | 5 | 5 |
| Erythritol (*2) (A) | 40 | 15 | 15 | 50 | 40 | 5 | 40 | 50 | 30 |
| Palatinit | 0 | 25 | 25 | 0 | 0 | 0 | 0 | 0 | 0 |
| Polyethylene glycol | 5 | 5 | 5 | 5 | 5 | 5 | 5 | 5 | 5 |
| Sodium carboxymethylcellul ose | 1 | 1 | 1 | 1 | 1 | 1 | 1 | 1 | 1 |
| Titanium oxide | 0.5 | 0.5 | 0.5 | 0.5 | 0.5 | 0.5 | 0.5 | 0.5 | 0.5 |
| Abrasive silica (*3) | 5 | 5 | 5 | 5 | 5 | 5 | 5 | 5 | 5 |
| Thickening silica (*4) | 4 | 4 | 4 | 4 | 4 | 4 | 4 | 4 | 4 |
| Flavoring agent | 1 | 1 | 1 | 1 | 1 | 1 | 1 | 1 | 1 |
| Sodium chloride | - | - | - | - | - | - | - | - | 15 |
| pH adjusting agent | Trace | Trace | Trace | Trace | Trace | Trace | Trace | Trace | Trace |
| Total | 100 | 100 | 100 | 100 | 100 | 100 | 100 | 100 | 100 |
| Water (C) | 15.8 | 15.6 | 16 | 12.9 | 16 | 24.8 | 15.3 | 12.9 | 12.9 |
| A/B | 200 | 30 | 150 | 250 | - | 25 | 57 | 500 | 500 |
| D/B | 7.5 | 3 | 15 | 7.5 | - | 7.5 | 2.1 | 15 | 15 |
| Foam fineness | 1 | 1 | 1 | 1 | 0 | 1 | 1 | 1 | -1 |
| Foam retention property | 1 | 1 | 1 | 1 | 0 | 1 | 1 | -1 | -1 |
| specific gravity 2/specific gravity 1 (Ratio of specific gravities) | 1.38/1.38 | 1.38/1.38 | 1.38/1.38 | 1.38/1.38 | 1.38/1.38 | 1.31/1.37 | 1.30/1.38 | 1.38/1.38 | 1.38/1.38 |
| | 1 | 1 | 1 | 1 | 1 | 0.95 | 0.94 | 1 | 1 |
| Defoaming property | good | good | good | good | good | poor | poor | good | good |

| | | | | | | | | | |
|---|---|---|---|---|---|---|---|---|---|
| (*1) AMISOFT MS-11 (AJINOMOTO CO., INC.) (*2) Average particle size: 200 µm (*3) Oil absorption capacity: 95 mL/100 g (*4) Oil absorption capacity: 310 mL/100 g | | | | | | | | | |

As shown in Table 2, in Examples 1 to 4, the dentifrice compositions of the present invention were found to form fine foam and to have a good foam retention property and a good defoaming property compared with the composition of Comparative Example 11 containing no sodium N-myristoyl glutamate . On the other hand, the composition of Comparative Example 13 containing 0.7 mass% of sodium N-myristoyl glutamate and the composition of Comparative Example 12 containing erythritol in an amount smaller than sodium N-myristoyl glutamate were found to have a defoaming property (specific gravity 2/specific gravity 1) of 0.95 and to be hardly defoamed. In addition, the composition of Comparative Example 14 having an (erythritol content/sodium N-myristoyl glutamate content) of 500 was found to be poor in foam retention property and to have a bad feeling upon use. Further, the composition of Comparative Example 15 containing sodium chloride was found to be poor in foam fineness and foam retention property and to have a bad feeling upon use.

The formulation of the dentifrice composition of the present invention is shown below. The dentifrice composition of Example 5 was found to form fine foam and to be satisfactory in foam retention property.

**Example 5**

| | |
|---|---|
| Erythritol (*1) | 45.0 |
| Sorbitol solution (70%) | 25.0 |
| Sodium N-lauroyl-L-glutamate | 0.2 |
| Sodium lauryl sulfate | 1.0 |
| Glycerin (98%) | 4.0 |
| Abrasive silica (*2) | 8.0 |
| Thickening Silica (*3) | 7.0 |
| Sodium carboxymethylcellulose | 0.5 |
| Xanthan gum | 0.05 |
| Carrageenan | 0.1 |
| Saccharin sodium | 0.04 |
| Titanium oxide | 0.5 |
| Sodium fluoride | 0.2 |
| Flavoring agent | 0.1 |
| Purified water | 8.31 |
| Total | 100.0 (mass%) |

| | |
|---|---|
| (*1) Average particle size: 200 µm (*2) Oil absorption capacity: 95 mL/100 g (*3) Oil absorption capacity: 310 mL/100 g | |

## Claims

1. A dentifrice composition, comprising the following components (A), (B), and (C) :
(A) 10 to 65 mass% of erythritol;
(B) 0.05 to 0.55 mass% of an N-acyl acidic amino acid or a salt thereof; and
(C) 10 to 25 mass% of water,
wherein a mass ratio (A/B) of the component (A) to the component (B) is 20 to 400, the content of sodium chloride in the dentrifice composition is 0.1 mass% or less, and an acyl group constituting the N-acyl acidic amino acid as the component (B) has 8 to 16 carbon atoms.

2. The dentifrice composition according to claim 1, wherein a mass ratio (A/C) of the component (A) to the component (C) is 0.7 to 6.

3. The dentifrice composition according to claim 1 or 2, wherein an acidic amino acid constituting the N-acyl acidic amino acid as the component (B) is glutamic acid.

4. The dentifrice composition according to any one of claims 1 to 3, further comprising 0.1 to 2.0 mass% of an alkyl sulfate salt as a component (D), wherein a mass ratio (D/B) of the component (D) to the component (B) is 0.2 to 20.

5. The dentifrice composition according to any one of claims 1 to 4, wherein the component (A) comprises 10 to 55 mass% of particulate erythritol having an average particle size of 350 µm or less.

6. The dentifrice composition according to any one of claims 1 to 5, wherein the mass ratio (A/B) of the component (A) to the component (B) is 20 to 250.

7. The dentifrice composition according to any one of claims 1 to 6, wherein the mass ratio (A/C) of the component (A) to the component (C) is 0.9 to 4.

8. The dentifrice composition according to any one of claims 1 to 7, wherein the mass ratio (D/B) of the component (D) to the component (B) is 2 to 15.

9. The dentifrice composition according to any one of claims 1 to 8, wherein a content of the water as the component (C) is 10 to 20 mass%.

10. The dentifrice composition according to any one of claims 1 to 9, further comprising 0 to 35 mass% of sorbitol and/or maltitol.

11. The dentifrice composition according to any one of claims 1 to 10, further comprising 0 to 8 mass% of glycerin.

12. The dentifrice composition according to any one of claims 1 to 10, wherein the content of sodium chloride in the dentrifice composition is 0.01 mass% or less.

13. The dentifrice composition according to any one of claims 1 to 11 for use in a method of removing a biofilm in an oral cavity.

## Patentansprüche

1. Zahnputzmittelzusammensetzung, umfassend die nachstehenden Komponenten (A), (B), und (C):
(A) 10 bis 65 Masse-% Erythrit;
(B) 0,05 bis 0,55 Masse-% einer sauren N-Acylaminosäure oder eines Salzes davon; und
(C) 10 bis 25 Masse-% Wasser,
wobei das Masseverhältnis (A/B) von Komponente (A) zu Komponente (B) 20 zu 400 beträgt, der Gehalt an Natriumchlorid in der Zahnputzmittelzusammensetzung 0,1 Masse-% oder weniger beträgt und eine Acylgruppe, die die saure N-Acylaminosäure als Komponente (B) bildet, 8 bis 16 Kohlenstoffatome aufweist.

2. Zahnputzmittelzusammensetzung gemäss Anspruch 1, wobei das Masseverhältnis (A/C) von Komponente (A) zu Komponente (C) 0,7 zu 6 beträgt.

3. Zahnputzmittelzusammensetzung gemäss Anspruch 1 oder 2, wobei eine saure Aminosäure, die die saure N-Acylaminosäure als Komponente (B) bildet, Glutaminsäure ist.

4. Zahnputzmittelzusammensetzung gemäss irgendeinem der Ansprüche 1 bis 3, die ferner 0,1 bis 2,0 Masse-% eines Alkylsulfatsalzes als Komponente (D) umfasst, wobei das Masseverhältnis (D/B) von Komponente (D) zu Komponente (B) 0,2 zu 20 beträgt.

5. Zahnputzmittelzusammensetzung gemäss irgendeinem der Ansprüche 1 bis 4, wobei die Komponente (A) 10 bis 55 Masse-% partikelförmiges Erythrit mit einer durchschnittlichen Partikelgrösse von 350 µm oder weniger umfasst.

6. Zahnputzmittelzusammensetzung gemäss irgendeinem der Ansprüche 1 bis 5, wobei das Masseverhältnis (A/B) von Komponente (A) zu Komponente (B) 20 zu 250 beträgt.

7. Zahnputzmittelzusammensetzung gemäss irgendeinem der Ansprüche 1 bis 6, wobei das Masseverhältnis (A/C) von Komponente (A) zu Komponente (C) 0,9 zu 4 beträgt.

8. Zahnputzmittelzusammensetzung gemäss irgendeinem der Ansprüche 1 bis 7, wobei das Masseverhältnis (D/B) von Komponente (D) zu Komponente (B) 2 zu 15 beträgt.

9. Zahnputzmittelzusammensetzung gemäss irgendeinem der Ansprüche 1 bis 8, wobei der Gehalt an Wasser als Komponente (C) 10 bis 20 Masse-% beträgt.

10. Zahnputzmittelzusammensetzung gemäss irgendeinem der Ansprüche 1 bis 9, die ferner 0 bis 35 Masse-% Sorbit und/oder Maltit umfasst.

11. Zahnputzmittelzusammensetzung gemäss irgendeinem der Ansprüche 1 bis 10, die ferner 0 bis 8 Masse-% Glycerin umfasst.

12. Zahnputzmittelzusammensetzung gemäss irgendeinem der Ansprüche 1 bis 10, wobei der Gehalt an Natriumchlorid in der Zahnputzmittelzusammensetzung 0,01 Masse-% oder weniger beträgt.

13. Zahnputzmittelzusammensetzung gemäss irgendeinem der Ansprüche 1 bis 11 zur Verwendung in einem Verfahren zur Entfernung eines Biofilms in der Mundhöhle.

## Revendications

1. Composition de pâte dentifrice, comprenant les composant (A), (B) et (C) suivants :
(A) de 10 à 65 % en masse d'érythritol ;
(B) de 0,05 à 0,55 % en masse d'un acide aminé acide N-acylé ou d'un sel de celui-ci ; et
(C) de 10 à 25 % en masse d'eau,
dans laquelle un rapport de masse (A/B) du composant (A) au composant (B) est de 20 à 400, la teneur en chlorure de sodium dans la composition de pâte dentifrice est de 0,1 % en masse ou moins, et un groupe acyle constituant l'acide aminé acide N-acylé comme composant (B) a de 8 à 16 atomes de carbone.

2. Composition de pâte dentifrice selon la revendication 1, dans laquelle un rapport de masse (A/C) du composant (A) au composant (C) est de 0,7 à 6.

3. Composition de pâte dentifrice selon la revendication 1 ou 2, dans laquelle un acide aminé acide constituant l'acide aminé acide N-acylé comme composant (B) est l'acide glutamique.

4. Composition de pâte dentifrice selon l'une quelconque des revendications 1 à 3, comprenant en outre de 0,1 à 2,0 % en masse d'un sel de sulfate d'alkyle comme composant (D), dans laquelle un rapport de masse (D/B) du composant (D) au composant (B) est de 0,2 à 20.

5. Composition de pâte dentifrice selon l'une quelconque des revendications 1 à 4, dans laquelle le composant (A) comprend de 10 à 55 % en masse d'érythritol particulaire ayant une taille de particule moyenne de 350 µm ou moins.

6. Composition de pâte dentifrice selon l'une quelconque des revendications 1 à 5, dans laquelle le rapport de masse (A/B) du composant (A) au composant (B) est de 20 à 250.

7. Composition de pâte dentifrice selon l'une quelconque des revendications 1 à 6, dans laquelle le rapport de masse (A/C) du composant (A) au composant (C) est de 0,9 à 4.

8. Composition de pâte dentifrice selon l'une quelconque des revendications 1 à 7, dans laquelle le rapport de masse (D/B) du composant (D) au composant (B) est de 2 à 15.

9. Composition de pâte dentifrice selon l'une quelconque des revendications 1 à 8, dans laquelle une teneur de l'eau comme composant (C) est de 10 à 20 % en masse.

10. Composition de pâte dentifrice selon l'une quelconque des revendications 1 à 9, comprenant en outre de 0 à 35 % en masse de sorbitol et/ou de maltitol.

11. Composition de pâte dentifrice selon l'une quelconque des revendications 1 à 10, comprenant en outre de 0 à 8 % en masse de glycérine.

12. Composition de pâte dentifrice selon l'une quelconque des revendications 1 à 10, dans laquelle la teneur en chlorure de sodium dans la composition de pâte dentifrice est de 0,01 % en masse ou moins.

13. Composition de pâte dentifrice selon l'une quelconque des revendications 1 à 11 à utiliser dans un procédé d'élimination d'un biofilm dans une cavité buccale.
